# EUROPEAN PATENT SPECIFICATION

(11) **EP 4 231 957 B1**
(45) Date of publication and mention of the grant of the patent: **11.02.2026**
(21) Application number: 21824237.8
(22) Date of filing: 01.12.2021
(51) Int. Cl.: A61B 90/00, A61B 17/00, A61B 17/02

(54) **A MARKER DELIVERY DEVICE CONFIGURED TO DECOUPLE PLUNGER AND PUSH ROD**
MARKERFREISETZUNGSVORRICHTUNG ZUR ENTKOPPLUNG VON KOLBEN UND SCHUBSTANGE
DISPOSITIF DE DISTRIBUTION DE MARQUEUR CONFIGURÉ POUR DÉCOUPLER UN PISTON ET UNE TIGE DE POUSSÉE

(30) Priority: 02.12.2020 US 202063120474 P
(43) Date of publication of application: 30.08.2023
(73) Proprietor: Devicor Medical Products, Inc., Cincinnati, OH 45241 (US)
(72) Inventor: MADAN, Ashvani, Mason, OH 45040 (US)
(74) Representative: Simmons & Simmons
(86) International application number: PCT/US2021/061386
(87) International publication number: WO 2022/119911

(56) References cited:
- EP-A2- 3 000 498
- WO-A1-2017/040064
- DE-A1- 102015 003 601

## Description

### PRIORITY

This application claims priority to U.S. Provisional Application Serial No. 63/120,474, entitled "Biopsy Deployer with Automatic Plunger Retraction," filed on December 2, 2020.

### TECHNICAL FIELD OF THE INVENTION

The invention relates to a marker delivery device for delivering a marker to a biopsy site in order to mark said biopsy site.

### BACKGROUND

A number of patients will have breast biopsies because of irregular mammograms and palpable abnormalities. Biopsies can include surgical excisional biopsies and stereotactic and ultrasound guided needle breast biopsies. In the case of an image directed biopsy, the radiologist or other physician may take a small sample of the irregular tissue for laboratory analysis. If the biopsy proves to be malignant, additional surgery (e.g., a lumpectomy or a mastectomy) may be required. In the case of needle biopsies, the patient may return to the radiologist a day or more later, and the biopsy site (the site of the lesion) may need to be relocated in preparation for the surgery. An imaging system, such as ultrasound, magnetic resonance imaging (MRI) or x-ray may be used to locate the biopsy site. In order to assist the relocation of the biopsy site, a marker may be placed at the time of the biopsy.

Markers are described in the following US Patents: US 6,083,524, "Polymerizable Biodegradable Polymers Including Carbonate or Dioxanone Linkages," issued July 4, 2000; US 6,162,241, "Hemostatic Tissue Sealants," issued December 4, 2000; US 6,270,464, "Biopsy Localization Method and Device," issued August 7, 2001; US 6,356,782, "Subcutaneous Cavity Marking Device and Method," issued March 12, 2002; US 6,605,294, "Methods of Using In Situ Hydration of Hydrogel Articles for Sealing or Augmentation of Tissue or Vessels," issued August 12, 2003; US 8,600,481, "Subcutaneous Cavity Marking Device," issued December 3, 2013 and US 8,939,910, "Method for Enhancing Ultrasound Visibility of Hyperechoic Materials", issued January 27, 2015.

In some contexts, a marker deployer requires an operator to press a plunger or plunger to translate a marker through a cannula using a push rod. Once the plunger is pressed, the push rod may protrude from a lateral aperture of a needle. After the marker is deployed, but before removing the deployer, the operator must release the plunger. The step of releasing the plunger, to some operators, is not always intuitive. As a result, a sharp edge of the lateral aperture or other surfaces associated with the biopsy device can shear the tip of the push rod leaving foreign objects in the body.
EP3 000 498 discloses an autoinjector which automatically extends a needle and injects a medicament in response to activation of a trigger.
WO2017/040064 discloses a tissue excision device with a coring cannula that extends distally from a handle along a longitudinal axis. A guide element being guided inside said cannula is connected with a plunger and also provides a locking member on its distal end. In order to mark tissue, a part of a locking member can be left inside tissue and serves as a marker.
DE 10 2015 003 601 discloses a biopsy instrument and handle for controlling deployment.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements. In the drawings, some components or portions of components are shown in phantom as depicted by broken lines.
FIGS. 1A, 1B, and 1C show exemplary aspects of placement of a biopsy site marker, in accordance with aspects of the present disclosure;
FIG. 2 depicts a perspective view of an exemplary marker delivery device;
FIG. 3 depicts a side cross-sectional view of the marker delivery device of FIG. 2;
FIG. 4 depicts a cross-sectional view of a marker being deployed from the distal portion of the marker delivery device of FIG. 1, and through a lateral aperture in a biopsy needle to mark a biopsy site;
FIG. 5 depicts a perspective view of another exemplary marker delivery device;
FIG. 6 depicts an enlarged perspective view of a plunger and a proximal end of a push rod assembly;
FIG. 7 depicts a partial perspective cross-sectional view of a handle, with the cross-section taken along line 7-7 of FIG. 5;
FIG. 8A depicts a partial side cross-sectional view of the marker delivery device of FIG. 5 with the cross-section taken along line 7-7 with the plunger and push rod assembly in an undeployed state;
FIG. 8B depicts another partial side cross-sectional view of the marker delivery device of FIG. 5 with the cross-section taken along line 7-7 with the plunger in a partially deployed state and the push rod assembly in a deployed state;
FIG. 8C depicts yet another partial side cross-sectional view of the marker delivery device of FIG. 5 with the cross-section taken along line 7-7 with the plunger in a deployed state and the push rod assembly in a partially retracted state; and
FIG. 8D depicts still another partial side cross-sectional view of the marker delivery device of FIG. 5 with the cross-section taken along line 7-7 with the plunger in deployed state and the push rod assembly in a retracted state.

The drawings are not intended to be limiting in any way, and it is contemplated that various embodiments of the invention may be carried out in a variety of other ways, including those not necessarily depicted in the drawings. The accompanying drawings incorporated in, and forming a part of the specification, illustrate several aspects of the present invention, and together with the description serve to explain the principles of the invention; it being understood, however, that this invention is not limited to the precise arrangements shown.

### DETAILED DESCRIPTION

The invention is defined by independent claim 1. Other examples, features, aspects. embodiments, and advantages will become apparent to those skilled in the art from the following description. Accordingly, the drawings and descriptions should be regarded as illustrative in nature and not restrictive.

It may be beneficial to be able to mark the location or margins of a lesion, whether temporarily or permanently, prior to or immediately after removing or sampling it. Marking prior to removal may help to ensure that the entire lesion is excised, if desired. Alternatively, if the lesion were inadvertently removed in its entirety, marking the biopsy site immediately after the procedure would enable reestablishment of its location for future identification.

Once a marker is positioned at a biopsy site, it may be desirable for the marker to remain visible under ultrasound. It may also be desirable to make the marker readily identifiable relative to other structural features of a patient. For instance, it may be desirable for the marker to be distinguishable under ultrasound visualization from microcalcifications to avoid inadvertently characterizing the marker as a microcalcification during subsequent ultrasonic examinations. Generally, microcalcifications are used in the field to identify suspicious lesions or masses. Thus, it is generally desirable for the ultrasound view to be distinguishable as a marker and not inadvertently identified as a new mass.

### I. Exemplary Marker

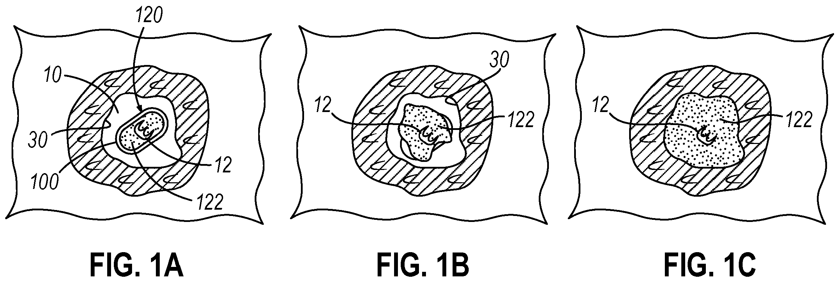

Aspects presented herein relate to devices and procedures for manufacturing a marker for percutaneously marking a biopsy cavity (10) having surrounding tissue (30), as shown in FIGS. 1A-1C. For instance, as seen in FIG. 1A, a marker (100) may be initially placed in the biopsy cavity (10) to facilitate relocation of the biopsy site. Marker (100) may comprise a carrier (120) and a marker element (12). Carrier (120) generally includes a bioabsorbable marker material (122). Thus, carrier (120) is generally configured for absorption into a patient after placement of marker (100) within the biopsy cavity (10). In some examples, carrier (120) can include a plurality of microbubbles to enhance visualization of carrier (120) under ultrasound. As will be described in greater detail below, marker material (122) is generally bioabsorbable such that marker material (122) may be generally absorbed into the patient's tissue over time. In the present example, marker material (122) comprises a hydrogel that is initially in a dehydrated state. Although a hydrogel is used in the present example, it should be understood that in other examples marker material (122) may comprise other known bioabsorbable materials.

In the present example, marker (100) further includes marker element (12) that is generally not bioabsorbable. Marker element (12) may comprise a radiopaque or echogenic marker embedded within the bioabsorbable marker material (122) of carrier (120). For instance, marker element (12) may comprise metal, hard plastic, or other radiopaque or hyperechoic materials known to those of ordinary skill in the art in view of the teachings herein. In other examples, marker (100) may be formed without marker element (12). In still other examples, marker (100) may be formed with only marker element (12) such that carrier (120) is omitted and marker element (12) is in a "bare" form. In other words, in some examples, marker (100) is formed of only carrier (120) as a bare clip.

Marker material (122) is generally expandable once disposed within a patient at a biopsy site. As shown in FIGS. 1B and 1C, the initially dehydrated marker material (122) may absorb fluid from the surrounding tissue (30) into which it is inserted. In response to this absorption of fluid, marker material (122) may swell, thereby permitting carrier (120) to fill a cavity formed at a biopsy site by removal of tissue samples during a biopsy procedure. Biodegradable materials may be particularly suitable in applications where it is desired that natural tissue growth be permitted to completely or partially replace the implanted material over time. Accordingly, biocompatibility is ensured, and the natural mechanical parameters of the tissue are substantially restored to those of the pre-damaged condition.

Marker (100) may be inserted into the body either surgically via an opening in the body cavity tissue (30), or through a minimally invasive procedure using such devices as a catheter, introducer or similar type insertion device. Marker (100) may be delivered immediately after removal of the tissue specimen using the same device used to remove the tissue specimen itself. Follow-up noninvasive detection techniques, such as x-ray mammography or ultrasound may then be used by the physician to identify, locate, and monitor the biopsy cavity site over a period of time via marker (100).

Marker (100) of the present example is large enough to be readily visible to a clinician under x-ray or ultrasonic viewing, for example; yet small enough to be able to be percutaneously deployed into the biopsy cavity and to not cause any difficulties with the patient. Although examples are described in connection with treatment and diagnosis of breast tissue, aspects presented herein may be used for markers in any internal tissue, e.g., in breast tissue, lung tissue, prostate tissue, lymph gland tissue, etc.

The hydration of the marker material (122) of carrier (120) by the natural moisture of the tissue surrounding it causes expansion of the polymer and thus minimizes the risk of migration. The growing hydrogel-based marker material (122) centers marker (100) in the biopsy cavity as it grows. As the hydrogel expands, naturally present moisture from the surrounding tissue, the hydration enables increasing sound through transmission, appears more and more hypoechoic and is easy to visualize on follow up ultrasound studies.

The hydrated hydrogel marker material (122) of carrier (120) may also be used to frame permanent marker (12). The hypoechoic nature of the hydrated marker material (122) enables ultrasound visibility of the permanent marker (12) within the hydrogel hydrated marker material (122) because the permanent marker (12) is outlined as a specular reflector within a hypoechoic hydrated marker having a waterlike nonreflective substrate.

### II. Exemplary Marker Delivery Device

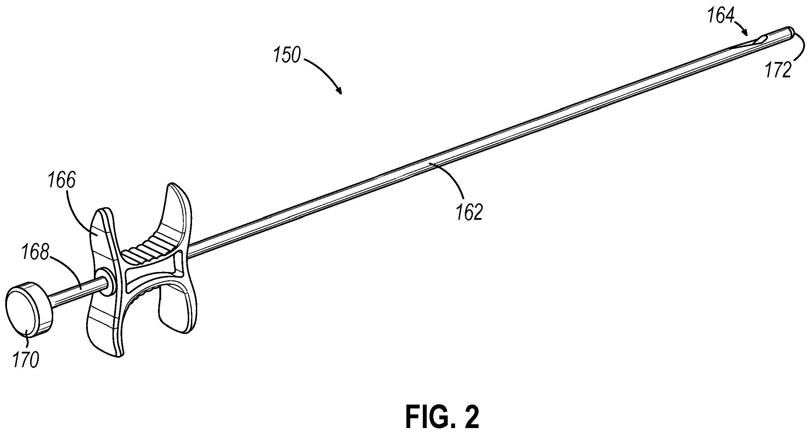

In some examples it may be desirable to deploy marker (100) described above within the body cavity tissue (30) using certain marker delivery devices. For instance, FIGS. 2 shows an exemplary marker delivery device (150) which includes an elongate outer cannula (162) having a marker exit, such as side opening (164) formed adjacent to, but spaced proximally from, the distal end of the cannula (162).

A grip (166) can be provided at the proximal end of cannula (162). A push rod (168) can be provided, with push rod (168) extending coaxially in cannula (162) such that push rod (168) is configured to translate within cannula (162) to displace one or more markers through side opening (164). Rod (168) may have sufficient rigidity in compression to push marker from an internal lumen (165) of cannula (162) out through opening (164) yet be relatively flexible in bending. A plunger (170) is coupled at the proximal end of rod (168) for forcing rod (168) distally in cannula (162) to deploy marker out of cannula (162).

An operator may grasp grip (166) with two fingers and may push on plunger (170) using the thumb on the same hand, so that marker delivery device (150) is operated by an operator's single hand. A spring (not shown) or another feature may be provided about rod (168) to bias rod (168) proximally relative to grip (166) and cannula (162).

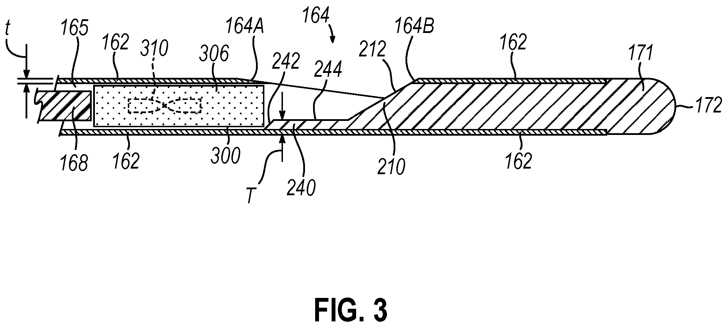

FIG. 3 shows a cross-sectional view of a distal portion of the marker delivery device (160). As can be seen, a biopsy marker (300) similar to marker (100) described above is disposed within internal lumen (165) of cannula (162). In the present example, marker (300) comprises a biodegradable or otherwise resorbable marker material (306), such as a generally cylindrically shaped body of collagen, hydrogel, etc., and a metallic, generally radiopaque permanent marker or marker element (310) (shown in phantom) disposed within or otherwise carried by marker material (306).

Cannula (162) may be formed of any suitable metallic or non-metallic material. In some versions, cannula (162) is formed of a thin walled hollow tube formed of a suitable medical grade plastic or polymer. One suitable material is a thermoplastic elastomer, such as Polyether Block Amide (PEBA), such as is known under the tradename PEBAX^{®}. Cannula (162) may be formed of PEBAX^{®} and may be substantially transparent to visible light and X-ray.

Side opening (164) may be formed by cutting away a portion of the wall of cannula (162). Side opening (164) communicates with an internal lumen (165) of cannula (162). Side opening (164) may extend axially (in a direction parallel to the axis of lumen (165)) from a proximal opening end (164A) to a distal opening end (164B) (see FIG. 3).

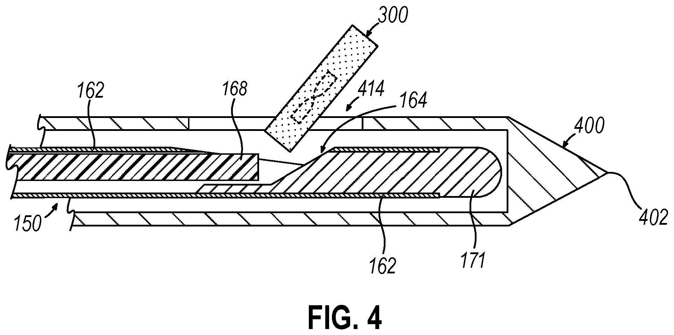

In the present example, distal tip (22) extends from the distal end of cannula (162) (see FIG. 2). The distal end of cannula (162) is closed by a unitary endpiece (171) (see FIG. 4), with a portion of endpiece (171) extending into internal lumen (165) of cannula (162). Endpiece (171) may be a molded or cast component. As shown in FIG. 3, endpiece (171) comprises a tip (172), a ramp (210) having a ramp surface (212), and a marker engaging element (240). Ramp surface (212) aids in directing marker (300) from internal lumen (165) through side opening (164). Marker engaging element (240) helps to retain marker (300) in internal lumen (165) until the operator intends to deploy marker (300).

Marker engaging element (240) is disposed within internal lumen (165), and at least a portion of marker engaging element (240) is disposed distally of proximal end (164A) of side opening (164). Marker engaging element (240) extends along a portion of the floor of cannula (162) under side opening (164) such that marker engaging element (240) is positioned to reinforce the portion of cannula (162) in which side opening (164) is formed. For instance, by positioning marker engaging element (240) underneath side opening (164), as shown in FIG. 3, marker engaging element (240) helps to stiffen cannula (162) in the region where wall of cannula (162) is cut to form side opening (164). As shown in FIG. 3, marker engaging element (240) extends from the proximal most portion of ramp surface (212) and does not extend proximally of side opening (164), though in other embodiments, a portion of marker engaging element (240) may extend proximally of opening (164).

As shown in FIG. 3, marker engaging element (240) is in the form of a step having a generally uniform thickness (T) along marker engaging element's (240) axial length, except that marker engaging element (240) has a tapered proximal end (242). Tapered proximal end (242) forms an included angle with the longitudinal axis of lumen (165) (included angle with a horizontal line in FIG. 3) of about 45 degrees, while ramp surface (212) forms an included angle with the longitudinal axis of about 30 degrees. Of course, any number of other suitable angles may be used.

As shown in FIG. 3, an upwardly facing surface (244) (surface facing opening (164)) of marker engaging element (240) extends distally to contact ramp surface (212), so that there is not a space or gap between surface (244) and ramp surface (212). Such an arrangement is advantageous to reduce the possibility that marker (300), upon moving past marker engaging element (240), may become lodged between marker engagement element (240) and ramp (212). In some versions, marker engaging element (240), ramp (210), and/or tip (172) are formed of, or include, a material that is relatively more radiopaque than the wall of cannula (162). For instance, where marker engaging element (240), ramp (210), and tip (172) are formed as an integral endpiece (171) (see FIG. 4), endpiece (171) may include a radiopaque additive, such as barium sulfate. For instance, endpiece (171) may be a component molded of PEBAX^{®}, with about 20 percent by weight barium sulfate added to the molten PEBAX^{®} mold composition. The relatively more radiopaque marker engaging element (240), ramp (210), and tip (172) may be useful in distinguishing the position of those components using radiographic imaging. Also, where ramp (210) and/or step of marker engaging element (240) are positioned in association with opening (164), the addition of a radiopaque material can help identify the position of opening (164), and the position of marker (300) relative to opening (164) before, during, or after deployment of marker (300).

Referring to FIG. 4, marker delivery device (150) (see FIG. 2) is used to deploy a marker (300) (see FIG. 3) to mark a biopsy location within a patient. In FIG. 4, a cannular biopsy needle (400) is shown having a closed distal end with piercing tip (402) and a lateral tissue receiving side aperture (414) Marker delivery device (150) (see FIG. 2) is introduced to a biopsy site through biopsy needle (400), which may be the same biopsy needle (400) used to collect a tissue sample from the biopsy site. Biopsy needle (400) may be of the type used with single insertion, multiple sample vacuum assisted biopsy devices. Several such biopsy devices are disclosed in the various patents and patent applications that have been referred to herein, though other biopsy devices may be used.

FIG. 4 shows the distal end of marker delivery device (150) (see FIG. 2) disposed within needle (400). Biopsy needle (400) may be positioned in tissue, and a biopsy sample may be obtained through lateral aperture (414), thereby providing a biopsy cavity adjacent lateral aperture (414). Then, after the tissue sample has been obtained and transferred proximally through biopsy needle (400), and without removing biopsy needle (400) from the patient's tissue, marker delivery device (150) is inserted into a proximal opening in biopsy needle (400). In FIG. 4, biopsy needle (400) and marker delivery device (150) are positioned such that opening (164) of cannula (162) and lateral aperture (414) of biopsy needle (400) are substantially aligned axially and circumferentially. Then, with marker delivery device (150) and biopsy needle (400) so positioned at the biopsy site, plunger (168) (see FIG. 3) is advanced to deploy marker (300) up ramp surface (212), through opening (164), and then through lateral aperture (414), into the biopsy cavity.

### III. Exemplary Marker Delivery Device Automatic Plunger Retraction

In some examples, it may be desirable to have a marker delivery device similar to marker delivery device (150) (see FIG. 2), that automatically retracts a plunger after an operator presses a plunger to deploy a marker. In particular, when an operator fully presses the plunger with a finger or a thumb, the plunger distally translates, pushing a marker through a side opening of a needle. When the plunger is distally translated, the plunger tip can protrude from side opening. If the operator inadvertently leaves their finger or thumb on the plunger, the plunger tip may remain protruded from side opening. If the operator removes the marker delivery device from a biopsy needle with plunger pressed, the protruding plunger tip can be severed through engagement against a sharp edge of the biopsy needle or cutter. After the plunger tip is sheared off, a portion of the plunger tip may remain in the patient's body. Thus, in some examples is may be desirable to have a marker delivery device that automatically translates the plunger proximally after marker deployment.

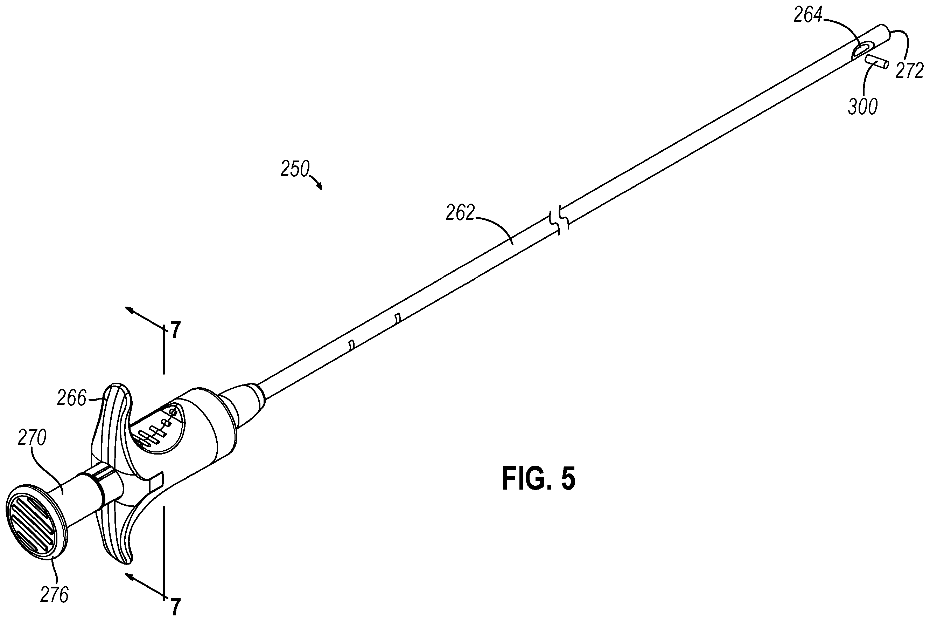

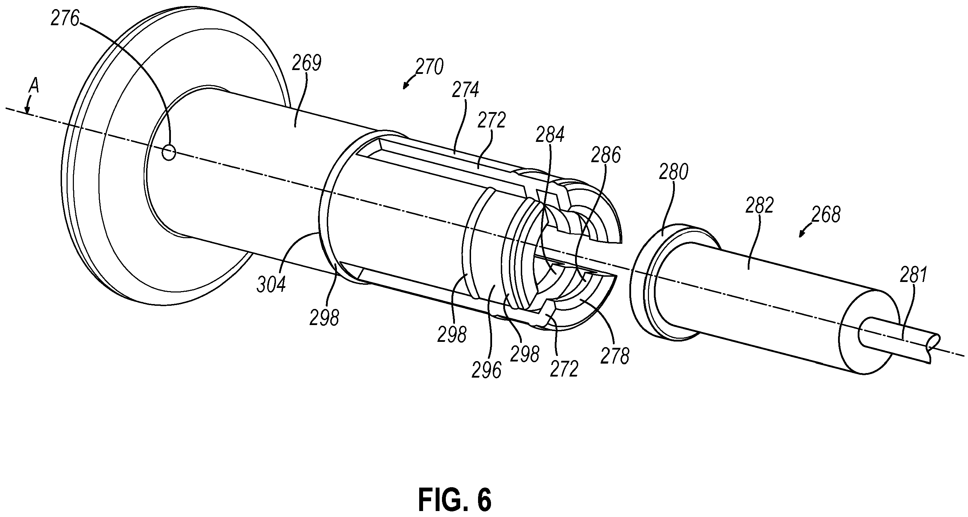

FIG. 5 shows an exemplary marker delivery device (250) that is generally configured to deploy a marker (300) through a side opening (264). Marker delivery device (250) is substantially similar to marker delivery device (150) described above except where explicitly noted herein. Like with marker delivery device (150), marker delivery device (250) has an elongate cannula (262), a grip (266), a plunger (270), a push rod assembly (268) (see FIG. 6) and a side opening (264). As with elongated cannula (162) described above, elongated cannula (262) of the present example extends distally from the grip (266). Grip (266) is configured for the operator to hold and manipulate marker delivery device (250). Plunger (270) is configured to translate marker (300) through side opening (264).

FIG. 6 shows plunger (270) and push rod assembly (268). However, unlike marker delivery device (150) that has plunger (170) fixed to push rod (168) (see FIG. 2), marker delivery device (250) (see FIG. 5) has plunger (270) removably coupled to push rod assembly (268). Plunger (270) includes one or more arms (274) and a vent (276). As will be described in greater detail below, arms (274) are generally configured to move relative to push rod assembly (268) to permit automatic retraction of push rod assembly (268) relative to plunger (270). Although plunger (270) of the present example includes three arms (274), it should be understood that in other examples, plunger (270) can be configured with any suitable number of arms (274).

Plunger (270) is hollow and includes a vent (276). Vent (276) can be located in the proximal end of plunger (270). Vent (276) can also be located in a proximal portion of plunger sidewall (269). Vent (276) enables egress of air through marker delivery device (250). Atmosphere is in communication with vent (276), such that air can pass through the hollow plunger (270) to elongate cannula (262). The elongated cannula (262) has a hollow around push rod (281). This hollow around push rod (281) allows air to exit the patient's body when marker (300) is deployed through side opening (264) of elongated cannula (262). Arms (274) are configured to transition between a biased state and an unbiased state. Arms (274) are shown in unbiased state. Arms (274) have one or more dogs (296) per arm (270) and one or more exterior bands (298) per arm (270). Dog (296) has a first rib (284) and a second rib (286). Dog (296) is configured to removably couple to at least a portion of push rod assembly (268). First rib (284) is located on a distal interior portion of dog (296) and proximally from second rib (286). Second rib (286) is spaced distally from first rib (284) and located on distal end of dog (296). First and second ribs (284, 286) are spaced apart in order to removably retain a portion of push rod assembly (268) between first and second ribs (284, 286). Second rib (286) has an angular surface (278) located on a distal interior portion of second rib (286). In other embodiments, a single rib (286) may engage two annular rings (280). In further embodiments, any number of ribs (286) may engage any number of annular rings (280).

Exterior bands (298) are longitudinally positioned on an exterior surface of arms (274) and an arm base (304). Exterior bands (298) are configured to engage a portion of the interior of the handle (266) (see FIG. 5) to provide tactile feedback to the operator.

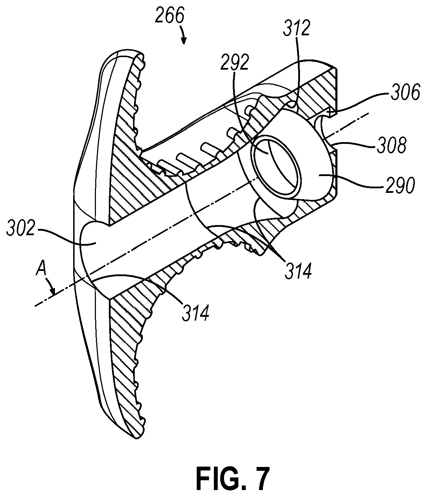

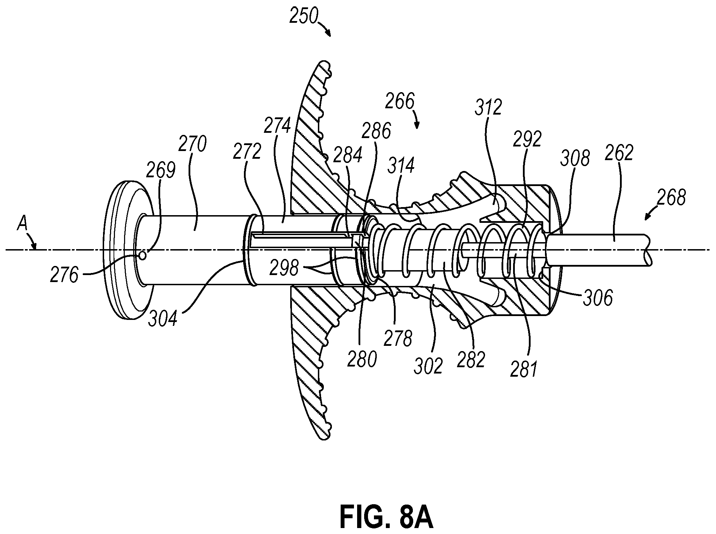

Plunger (268) includes annular ring (280), a barrel (282), and a push rod (281). Annular ring (280) is proximally located in relation to barrel (282). Annular ring (280) is larger in diameter than barrel (282). Barrel (282) is proximally located in relation to push rod (281). Barrel (282) is larger in diameter than push rod (281). Push rod (281) distally extends through second bore (308) (see FIG. 7) and further extends distally through elongated cannula (262) (see FIG. 8A) to a push rod distal tip (not shown). Push rod distal tip (not shown) is located distal in relation to side opening (264) (see Fig. 5).

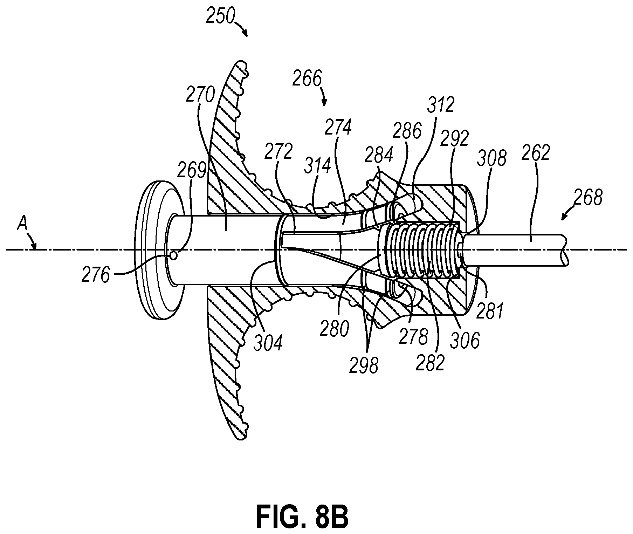

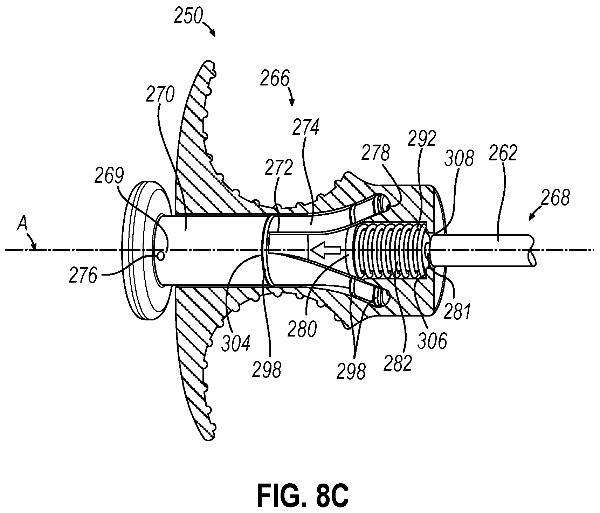

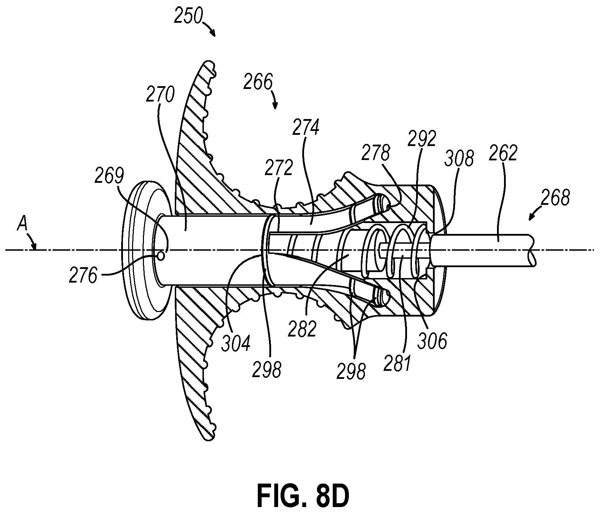

FIG. 7 shows a handle (266) including a first bore (302), a conical ring (290), a spring holding cavity (292) and a second bore (308). First bore (302) extends distally along longitudinal axis (A) from a proximal end of handle (266) to conical ring (290). At conical ring (290), first bore (302) diverges from longitudinal axis (A) into a first bore cavity (312). Spring holding cavity (292) is located within the conical ring (290). Spring holding cavity (292) extends distally from narrow portion of conical ring (290) to a proximal face (306) of second bore (308). Spring holding cavity (292) is configured to retain a spring (294) (see FIG. 8). Second bore (308) is distally located from spring holding cavity (292). Second bore (308) extends from proximal face (306) to distal end of handle (266). Second bore (308) has a smaller diameter than spring holding cavity (292).

Conical ring (290) has a ramp that includes a wide portion and a narrow portion. Narrow portion is proximally located relative to wide portion. Spring holding cavity (292) is located within conical ring (290). Conical ring (290) is configured to engage angular surface (278) (see FIG. 6) at the narrow portion and expand the distal end of arms (274) as angular surface (278) (see FIG. 6) rides along conical ring (290) to the wide portion.

First bore (302) and first bore cavity (312) have a plurality of interior bands (314). Interior bands (314) can have a variety of geometries. Generally, Interior bands (314) have any geometry complementary to the geometry of the exterior bands (298). Interior bands (314) can be passive grooves or protrusions. Interior bands (314) can have an arcuate shape, a triangular shape, or a rectangular shape. Interior bands (314) are located on an interior surface of first bore (302) and an interior surface of first bore cavity (312). Interior bands (314) are longitudinally spaced to give the operator tactile feedback at different stages of deployment. These stages of deployment can be but are not limited to an undeployed stage (see Fig. 8A), a partially deployed stage (see Fig. 8B), and a deployed stage (see Figs. 8C and 8D). Interior bands (314) are configured to engage exterior bands (298) (see FIG. 6) with enough resistance to give the operator tactile feedback, but not enough resistance to materially inhibit movement of plunger (270) and push rod assembly (268).

FIG. 8A shows marker delivery device (250) with push rod assembly (268) in an undeployed state. In the undeployed state, plunger (270) is removably coupled to push rod assembly (268). Arms (274) are in unbiased state with each arm (274) having a respective dog (296) engaging annular ring (280). One or more exterior bands (298) can engage one or more interior bands (314) to resist longitudinal movement of plunger (270). In the undeployed state, exterior bands (298) and interior bands (314) can resist longitudinal movement to keep the operator from inadvertently pushing plunger (270).

Spring (294) is located within spring holding cavity (292) between annular ring (280) and proximal face (306). Spring (294) depicted in FIG. 8A is a helical compression coil spring. Spring (294) is not limited to being configured as helical compression spring. A tension spring may also be used. However, a variety of other springs or other resilient member features can be used. Spring (294) may be any resilient member such as rubber, foam, or belleville washers. Spring (294) biases push rod assembly (268) proximally. Spring (294) is sized to be located around barrel (282) and within spring holding cavity (292).

FIG. 8B shows marker delivery device (250) with plunger (270) distally translating push rod assembly (268). To translate plunger (270), an operator can hold grip (266) and push the proximal end of plunger (270) with a thumb or a finger to thereby distally translate plunger (270). Dog (296) remains coupled to annular ring (280), which distally translates push rod assembly (268). Push rod assembly (268) continues to translate distally while plunger (270) is translated until distal end of barrel (282) engages proximal face (306). Barrel (282) is longitudinally sized to allow push rod assembly (268) to translate a specific longitudinal distance before push rod assembly (268) engages proximal face (306). The push rod assembly (268) engages proximal face (306) when angular surface (278) engages narrow portion of conical ring (290). Barrel (282) has a larger diameter than push rod (281). Smaller diameter push rod (281) translates through second bore (308) until barrel (282) stops at proximal face (306). Once barrel (282) engages proximal face (306), push rod assembly (268) stops moving distally, and at this point marker (300) (see FIG. 5) has been deployed through side opening (264) (see FIG. 5). Angular surface (278) of second rib (286) begins to engage conical ring (290). Conical ring (290) begins to deflect arms (274) away from longitudinal axis (A).

FIG. 8C shows marker delivery device (250) with plunger (270) in the deployed state and with plunger (270) distally translated. As plunger (270) is pushed distally, angular surfaces (278) engage narrow portion of conical ring (290) (see FIG. 8B) and angular surfaces (278) ride along narrow portion of conical ring (290) to wide portion of conical ring (290). Arms (274) are transitioned from an unbiased state (see FIG. 8B) to a biased state. Arms (274) are deflected from longitudinal axis (A). Each arm (274) moves radially about arm base (304) away from the longitudinal axis (A). In response to arms (274) being deflected, the interior diameter defined by the first rib (284) of each arm (274) becomes wider than an external diameter of annular ring (280), which allows wider interior diameter of first rib (284) to release annular ring (280). After dog (296) releases annular ring (280), spring (294) begins to proximally translate push rod assembly (268). Prior to dogs (296) being fully translated into first bore cavity (308), interior bands (314) engage exterior bands (298) giving the operator tactile feedback that plunger (270) is close to being fully depressed.

FIG. 8D shows marker delivery device (250) with plunger (270) fully translated distally, and push rod assembly (268) fully translated proximally. Spring (294) translates push rod assembly (268) proximally until spring (294) is no longer biased to expand, and spring returns to a relaxed state.

Some operators are accustomed to pressing plunger (270), on other marker delivery devices more than once to ensure marker delivery. In other embodiments, handle (266) can have a stop (not shown) to prevent push rod assembly (268) from moving proximally, once spring (294) has retracted push rod assembly (268). This stop (not shown) also keeps spring slightly biased between annular ring (280) (see FIG. 8C) and proximal face (306) of second bore (308). This slight bias on spring (294) can keep spring (294) aligned with longitudinal axis (A). In other embodiments, this stop (not shown) may be located on push rod (281). Stop (not shown) can be located distally in relation to second bore (308) and operatively coupled to push rod (281). This stop (not shown) is configured to inhibit longitudinal movement of push rod (281) through second bore (308) and thereby inhibiting spring (294) from translating push rod assembly (268) proximally. This stop (not shown) can aid the operator in returning plunger (270) and push rod assembly (268) to coupled state (see FIG. 8A) to press plunger (270) more than once. In order to reset plunger (270) and plunger (268) to the undeployed state (see FIG. 8A), the operator translates plunger (270) proximally, while holding handle (266). First rib (284) rides over annular ring (280) (see FIG 8C), returning annular ring (280) between first rib (284) and second rib (286). In such embodiments, first rib can have a tapered proximal edge (not shown) to aid the operator in retracting plunger (268) within inner bore of handle (266) to retain annular ring (280) between first rib (284) and second rib (286).

The invention is defined by the appended claims.

## Claims

1. A marker delivery device for delivering a marker to a biopsy site to mark the biopsy site, comprising:
(a) a handle (266) including a first bore (302) disposed within a portion of the handle;
(b) an elongate cannula (262) extending distally from the handle along a longitudinal axis;
(c) a push rod assembly (268) including a mating feature (280, 282), a portion of the push rod assembly being disposed within the first bore and extending distally through the elongate cannula; and
(d) a plunger (270),
**characterized in that** the plunger includes an engagement feature, a portion of the plunger being disposed within the first bore proximally of the push rod assembly, the engagement feature being removably coupled to the mating feature, and the engagement feature being configured to de-couple from the mating feature when the plunger translates the push rod assembly distally.

2. The marker delivery device of claim 1, the handle including a ramped surface (290) located distally in relation to the first bore, the engagement feature defining an angled surface (270), the ramped surface being configured to engage the angled surface to de-couple the engagement feature from the mating feature when the plunger is translated distally.

3. The marker delivery device of claim 2, further comprising a spring configured to bias the plunger proximally after the engagement feature de-couples from the mating feature.

4. The marker delivery device of any one or more of claims 1 through 3, the plunger including a vent aperture (276) and a hollow portion, the vent aperture being in a proximal end of the plunger, the vent aperture being in fluid communication with the hollow portion, the hollow portion being in fluid communication with an opening of the cannula.

5. The marker delivery device of any one or more of claims 2 through 4, the engagement feature including a first arm (274) biased at an arm base (304), the first arm being configured to radially deflect at an arm base away from the longitudinal axis when the first arm engages the ramped surface.

6. The marker delivery device of claim 5, the first arm including a first rib (284) configured to engage the mating feature.

7. The marker delivery device of claim 6, the first arm having a plurality of ribs (284, 286) configured to engage the mating feature.

8. The marker delivery device of any one or more of claims 1 through 7, the mating feature defining a first annular ring (280).

9. The marker delivery device of any one or more of claims 1 through 8, the mating feature defining a plurality of annular rings (280).

10. The marker delivery device of any one or more of claims 3 through 7, the ramped surface including a spring bore (292) disposed within a portion of the ramped surface, the spring bore being configured to retain the spring.

11. The marker delivery device of claim 10, the handle including a second bore (308) distally located from the spring bore, and a proximal face (306) located between the spring bore and the second bore, the push rod assembly further including a barrel (282) and a push rod (281), the barrel being configured to engage the face.

12. The marker delivery device of claim 11, the barrel being configured to stop the longitudinal movement of plunger when the barrel engages the proximal face when the angled surface engages the ramped surface.

13. The marker delivery device of any one or more of claims 1 through 12, the handle including a plurality of internal surface effects disposed transverse to the longitudinal axis on an interior surface of the first bore, the plunger including a plurality of external surface effects displaced longitudinally along an exterior surface of the plunger, the external surface effects being configured to engage the internal surface effect to give an operator tactile feedback when the plunger is located at predetermined longitudinal position.

14. The marker delivery device of claim 13, the internal surface effect being a raised portion and the external surface effect being a recessed portion.

15. The marker delivery device of claim 13, the internal surface effect being a recessed portion and the external surface effect being a raised portion.

## Patentansprüche

1. Markerfreisetzungsvorrichtung zum Freisetzen eines Markierungsmittels an eine Biopsiestelle, um die Biopsiestelle zu markieren, umfassend:
(a) einen Griff (266) einschließlich einer ersten Bohrung (302), die in einem Abschnitt des Griffs angeordnet ist;
(b) eine längliche Kanüle (262), die sich distal vom Griff entlang einer Längsachse erstreckt;
(c) eine Schubstangenanordnung (268) einschließlich eines Gegenstückmerkmals (280, 282), wobei ein Abschnitt der Schubstangenanordnung innerhalb der ersten Bohrung angeordnet ist und sich distal durch die längliche Kanüle hindurch erstreckt; und
(d) einen Kolben (270),
**dadurch gekennzeichnet, dass** der Kolben ein Eingriffnahmemerkmal einschließt, wobei ein Abschnitt des Kolbens innerhalb der ersten Bohrung proximal zur Schubstangenanordnung angeordnet ist, wobei die Eingriffnahmemerkmal lösbar mit dem Gegenstückmerkmal verbunden ist, und wobei das Eingriffnahmemerkmal so konfiguriert ist, dass es sich von dem Gegenstückmerkmal löst, wenn der Kolben die Schubstangenanordnung distal verschiebt.

2. Markerfreisetzungsvorrichtung nach Anspruch 1, wobei der Griff eine abgeschrägte Oberfläche (290) einschließt, die in Bezug auf die erste Bohrung distal gelegen ist, wobei das Eingriffnahmemerkmal eine abgewinkelte Oberfläche (270) definiert, wobei die abgeschrägte Oberfläche so konfiguriert ist, dass sie mit der abgewinkelten Oberfläche in Eingriff kommt, um das Eingriffnahmemerkmal von dem Gegenstückmerkmal zu entkoppeln, wenn der Kolben distal verschoben wird.

3. Markerfreisetzungsvorrichtung nach Anspruch 2, weiter umfassend eine Feder, die so konfiguriert ist, dass sie den Kolben proximal vorspannt, nachdem sich das Eingriffnahmemerkmal vom Gegenstückmerkmal entkoppelt hat.

4. Markerfreisetzungsvorrichtung nach einem oder mehreren der Ansprüche 1 bis 3, wobei der Kolben eine Entlüftungsöffnung (276) und einen hohlen Abschnitt einschließt, wobei sich die Entlüftungsöffnung an einem proximalen Ende des Kolbens befindet, wobei die Entlüftungsöffnung in Fluidverbindung mit dem hohlen Abschnitt steht, wobei der hohle Abschnitt in Fluidverbindung mit einer Öffnung der Kanüle steht.

5. Markerfreisetzungsvorrichtung nach einem oder mehreren der Ansprüche 2 bis 4, wobei das Eingriffnahmemerkmal einen ersten Arm (274) einschließt, der an einer Armbasis (304) vorgespannt ist, wobei der erste Arm so konfiguriert ist, dass er sich an einer Armbasis radial von der Längsachse weg auslenkt, wenn der erste Arm mit der abgeschrägten Oberfläche in Eingriff kommt.

6. Markerfreisetzungsvorrichtung nach Anspruch 5, wobei der erste Arm eine erste Rippe (284) einschließt, die so konfiguriert ist, dass sie mit dem Gegenstückmerkmal in Eingriff kommt.

7. Markerfreisetzungsvorrichtung nach Anspruch 6, wobei der erste Arm eine Vielzahl von Rippen (284, 286) aufweist, die so konfiguriert sind, dass sie mit dem Gegenstückmerkmal in Eingriff kommen.

8. Markerfreisetzungsvorrichtung nach einem oder mehreren der Ansprüche 1 bis 7, wobei das Gegenstückmerkmal einen ersten Kreisring (280) definiert.

9. Markerfreisetzungsvorrichtung nach einem oder mehreren der Ansprüche 1 bis 8, wobei das Gegenstückmerkmal eine Vielzahl von Kreisringen (280) definiert.

10. Markerfreisetzungsvorrichtung nach einem oder mehreren der Ansprüche 3 bis 7, wobei die abgeschrägte Oberfläche eine Federbohrung (292) einschließt, die innerhalb eines Abschnitts der abgeschrägten Oberfläche angeordnet ist, wobei die Federbohrung so konfiguriert ist, dass sie die Feder hält.

11. Markerfreisetzungsvorrichtung nach Anspruch 10, wobei der Griff eine zweite Bohrung (308) einschließt, die distal von der Federbohrung gelegen ist, und eine proximale Vorderseite (306), die zwischen der Federbohrung und der zweiten Bohrung gelegen ist, wobei die Schubstangenanordnung weiter einen Zylinder (282) und eine Schubstange (281) einschließt, wobei der Zylinder so konfiguriert ist, dass er mit der Vorderseite in Eingriff kommt.

12. Markerfreisetzungsvorrichtung nach Anspruch 11, wobei der Zylinder so konfiguriert ist, dass er die Längsbewegung des Kolbens stoppt, wenn der Zylinder mit der proximalen Vorderseite in Eingriff kommt, wenn die abgewinkelte Oberfläche mit der abgeschrägten Oberfläche in Eingriff kommt.

13. Markerfreisetzungsvorrichtung nach einem oder mehreren der Ansprüche 1 bis 12, wobei der Griff eine Vielzahl von inneren Oberflächeneffekten einschließt, die quer zur Längsachse auf einer inneren Oberfläche der ersten Bohrung angeordnet sind, wobei der Kolben eine Vielzahl von äußeren Oberflächeneffekten einschließt, die in Längsrichtung entlang einer äußeren Oberfläche des Kolbens versetzt sind, wobei die äußeren Oberflächeneffekte so konfiguriert sind, dass sie mit dem inneren Oberflächeneffekt in Eingriff kommen, um dem Bediener ein taktiles Feedback zu geben, wenn sich der Kolben an einer vorbestimmten Längsposition befindet.

14. Markerfreisetzungsvorrichtung nach Anspruch 13, wobei der innere Oberflächeneffekt ein erhabener Abschnitt und der äußere Oberflächeneffekt ein vertiefter Abschnitt ist.

15. Markerfreisetzungsvorrichtung nach Anspruch 13, wobei der innere Oberflächeneffekt ein vertiefter Abschnitt und der äußere Oberflächeneffekt ein erhabener Abschnitt ist.

## Revendications

1. Dispositif de distribution de marqueur pour administrer un marqueur à un site de biopsie pour marquer le site de biopsie, comprenant :
(a) une poignée (266) incluant un premier alésage (302) disposé dans une partie de la poignée ;
(b) une canule allongée (262) s'étendant distalement par rapport à la poignée le long d'un axe longitudinal ;
(c) un ensemble tige de poussée (268) incluant un élément d'accouplement (280, 282), une partie de l'ensemble tige de poussée étant disposée dans le premier alésage et s'étendant distalement à travers la canule allongée ; et
(d) un piston (270), **caractérisé en ce que** le piston inclut un élément de mise en prise, une partie du piston étant disposée dans le premier alésage proximalement à l'ensemble tige de poussée, l'élément de mise en prise étant couplé de manière amovible à l'élément d'accouplement, et l'élément de mise en prise étant configuré pour se découpler de l'élément d'accouplement lorsque le piston déplace par translation l'ensemble tige de poussée distalement.

2. Dispositif de distribution de marqueur selon la revendication 1, la poignée incluant une surface inclinée (290) située distalement par rapport au premier alésage, l'élément de mise en prise définissant une surface angulaire (270), la surface inclinée étant configurée pour être en prise avec la surface angulaire afin de découpler l'élément de mise en prise de l'élément d'accouplement lorsque le piston est déplacé en translation distalement.

3. Dispositif de distribution de marqueur selon la revendication 2, comprenant en outre un ressort configuré pour solliciter le piston de manière proximale après que l'élément de mise en prise s'est découplé de l'élément d'accouplement.

4. Dispositif de distribution de marqueur selon l'une quelconque ou plusieurs des revendications 1 à 3, le piston incluant un orifice d'évent (276) et une partie creuse, l'orifice d'évent étant dans une extrémité proximale du piston, l'orifice d'évent étant en communication fluidique avec la partie creuse, la partie creuse étant en communication fluidique avec une ouverture de la canule.

5. Dispositif de distribution de marqueur selon l'une quelconque ou plusieurs des revendications 2 à 4, l'élément de mise en prise incluant un premier bras (274) sollicité au niveau d'une base (304) de bras, le premier bras étant configuré pour dévier radialement au niveau d'une base de bras à l'écart de l'axe longitudinal lorsque le premier bras est en prise avec la surface inclinée.

6. Dispositif de distribution de marqueur selon la revendication 5, le premier bras incluant une première nervure (284) configurée pour être en prise avec l'élément d'accouplement.

7. Dispositif de distribution de marqueur selon la revendication 6, le premier bras présentant une pluralité de nervures (284, 286) configurées pour être en prise avec l'élément d'accouplement.

8. Dispositif de distribution de marqueur selon l'une quelconque ou plusieurs des revendications 1 à 7, l'élément d'accouplement définissant une première bague annulaire (280).

9. Dispositif de distribution de marqueur selon l'une quelconque ou plusieurs des revendications 1 à 8, l'élément d'accouplement définissant une pluralité de bagues annulaires (280).

10. Dispositif de distribution de marqueur selon l'une quelconque ou plusieurs des revendications 3 à 7, la surface inclinée incluant un alésage (292) à ressort disposé dans une partie de la surface inclinée, l'alésage à ressort étant configuré pour maintenir le ressort.

11. Dispositif de distribution de marqueur selon la revendication 10, la poignée incluant un second alésage (308) situé distalement par rapport à l'alésage à ressort, et une face proximale (306) située entre l'alésage à ressort et le second alésage, l'ensemble tige de poussée incluant en outre un cylindre (282) et une tige de poussée (281), le cylindre étant configuré pour être en prise avec la face.

12. Dispositif de distribution de marqueur selon la revendication 11, le cylindre étant configuré pour arrêter le mouvement longitudinal du piston lorsque le cylindre est en prise avec la face proximale lorsque la surface angulaire est en prise avec la surface inclinée.

13. Dispositif de distribution de marqueur selon l'une quelconque ou plusieurs des revendications 1 à 12, la poignée incluant une pluralité d'effets de surface interne disposés transversalement par rapport à l'axe longitudinal sur une surface intérieure du premier alésage, le piston incluant une pluralité d'effets de surface externe déplacés longitudinalement le long d'une surface extérieure du piston, les effets de surface externe étant configurés pour être en prise avec l'effet de surface interne afin de fournir un retour tactile à un opérateur lorsque le piston est situé à une position longitudinale prédéterminée.

14. Dispositif de distribution de marqueur selon la revendication 13, l'effet de surface interne étant une partie surélevée et l'effet de surface externe étant une partie en creux.

15. Dispositif de distribution de marqueur selon la revendication 13, l'effet de surface interne étant une partie en creux et l'effet de surface externe étant une partie surélevée.
